# EUROPEAN PATENT APPLICATION

(11) **EP 4 841 562 A1**
(43) Date of publication of application: **18.11.2026**
(21) Application number: 24917017.6
(22) Date of filing: 11.12.2024
(51) Int. Cl.: G06T 7/20, A61B 5/11, A61B 10/00, G06T 7/00

(54) **COGNITIVE FUNCTION EVALUATION SYSTEM**

(30) Priority: 12.01.2024 JP 2024003566
(71) Applicant: The University of Osaka, Suita-shi, Osaka 565-0871 (JP)
(72) Inventor: YAGI, Yasushi, Suita-shi, Osaka 565-0871 (JP); MAKIHARA, Yasushi, Suita-shi, Osaka 565-0871 (JP); WU, Shuqiong, Suita-shi, Osaka 565-0871 (JP); GODO, Akos, Suita-shi, Osaka 565-0871 (JP)
(74) Representative: KATZAROV S.A.
(86) International application number: PCT/JP2024/043773
(87) International publication number: WO 2025/150332

(57) **Abstract**

A cognitive function evaluation system (100) includes an imager (10), a generator (30), a preprocessor (40), and an evaluator (60). The imager (10) images a subject (SJ) to be evaluated performing a dual-task in which a cognitive task and a motor task are simultaneously assigned, and generates imaging signals. The generator (30) generates, based on the imaging signals, time-series data of a skeleton representing at least a part of a whole body of the subject (SJ) to be evaluated performing the dual-task. The preprocessor (40) decomposes the time-series data of the skeleton into periods to generate period-based skeleton data items, and aligns phases of the period-based skeleton data items to generate input data. The evaluator (60) extracts a motion feature of the subject (SJ) to be evaluated based on the input data, and evaluates a cognitive function of the subject (SJ) to be evaluated based on the motion feature of the subject (SJ) to be evaluated which has been extracted.

## Description

### [Technical Field]

The present invention relates to a cognitive function evaluation system.

### [Background Art]

Paper-based tests such as the Mini-Mental State Examination (MMSE) are typically used for evaluating cognitive functions. However, this type of paper-based test contains fixed questions. For this reason, when this type of test is routinely used, there is a possibility that a subject to be evaluated memorizes the content of the questions, which may result in a failure to correctly evaluate the cognitive functions of the subject. For this reason, there is a demand for a technique that is capable of routinely evaluating cognitive functions of subjects.

As one technique for evaluating cognitive functions, there is a technique that uses a dual-task in which a subject to be evaluated is simultaneously assigned two tasks (for example, Non-Patent Literatures (NPLs) 1 and 2). If cognitive functions can be evaluated by employing such a dual-task, cognitive functions of subjects can be routinely evaluated.

### [Citation List]

### [Non-Patent Literature]

[NPL 1] H. Makizako et al., "Relationship between dual-task performance and neurocognitive measures in older adults with mild cognitive impairment", Geriatr. Gerontol. Int., pp. 314-321, 2013.
[NPL 2] K. Aoki et al., "Early detection of lower mmse scores in elderly based on dual-task gait", IEEE Access, vol. 7, pp. 40085-40094, 2019.

### [Summary of Invention]

### [Technical Problem]

The inventors of the present application conducted extensive research into techniques for evaluating cognitive functions using a dual-task and have completed the present invention. The present invention provides a cognitive function evaluation system that can evaluate a cognitive function of a subject to be evaluated using a dual-task.

### [Solution to Problem]

According to one aspect of the present invention, a cognitive function evaluation system is a system that evaluates a cognitive function of a subject to be evaluated. The cognitive function evaluation system includes: an imager, a generator, a preprocessor, and an evaluator. The imager images the subject to be evaluated performing a dual-task in which a cognitive task and a motor task are simultaneously assigned, and generates imaging signals. The generator generates, based on the imaging signals, time-series data of a skeleton representing at least a part of a whole body of the subject to be evaluated performing the dual-task. The preprocessor decomposes the time-series data of the skeleton into periods to generate period-based skeleton data items, and aligns phases of the period-based skeleton data items to generate input data. The evaluator extracts a motion feature of the subject to be evaluated based on the input data, and evaluates a cognitive function of the subject to be evaluated based on the motion feature of the subject to be evaluated which has been extracted.

In one embodiment, the time-series data of the skeleton indicates time-series data of each of a plurality of joints included in the skeleton. The plurality of joints indicate at least part of all joints included in the skeleton. The preprocessor decomposes the time-series data of each of the plurality of joints into periods to generate period-based joint data items, and aligns phases of the period-based joint data items to generate the input data.

In one embodiment, from each of the period-based joint data items, the preprocessor samples a fixed number of data items of same phase points to generate the input data.

In one embodiment, for each of the plurality of joints, the preprocessor selects, from among the period-based joint data items, a fixed number of consecutive period-based joint data items having a highest correlation to generate the input data.

In one embodiment, the input data indicates the period-based joint data items whose phases are aligned. The period-based joint data items whose phases are aligned indicate spatial-dimension data, phase-dimension data, and period-dimension data. The evaluator extracts the motion feature of the subject to be evaluated by convolving each of the spatial-dimension data, the phase-dimension data, and the period-dimension data.

In one embodiment, the evaluator includes: a first convolutional layer and a second convolutional layer. The first convolutional layer convolves the phase-dimension data included in the input data. The second convolutional layer extracts the motion feature of the subject to be evaluated by convolving each of the spatial-dimension data, the phase-dimension data, and the period-dimension data included in the input data obtained after the phase-dimension data is convolved by the first convolutional layer.

In one embodiment, the evaluator further includes a multiplier. The multiplier multiplies an adjacency matrix indicating positional relationships of the plurality of joints by an output of the first convolutional layer. An output of the multiplier is input to the second convolutional layer.

In one embodiment, the evaluator further includes a residual neural network that performs operation on a difference between an output of the second convolutional layer and the input data.

In one embodiment, the evaluator includes a plurality of convolutional blocks connected in series. Each of the plurality of convolutional blocks convolves the spatial-dimension data, the phase-dimension data, and the period-dimension data.

In one embodiment, the above-described cognitive function evaluation system further includes an answer detector that detects an answer given by the subject to be evaluated to a cognitive task. The answer detector detects at least one of (i) an answer given by the subject to be evaluated performing the dual-task or (ii) an answer given by the subject to be evaluated performing a cognitive task in which a cognitive task is assigned. The evaluator further extracts an answer feature of the answer detected by the answer detector, and evaluates the cognitive function of the subject to be evaluated, based on the motion feature and the answer feature of the subject to be evaluated.

### [Advantageous Effects of Invention]

A cognitive function evaluation system according to the present invention can evaluate the cognitive function of a subject to be evaluated using a dual-task.

### [Brief Description of Drawings]

[FIG. 1]
   A diagram illustrating a cognitive function evaluation system according to an embodiment of the present invention.
[FIG. 2]
   A block diagram illustrating a hardware configuration of the cognitive function evaluation system according to the embodiment of the present invention.
[FIG. 3]
   Parts (a) to (d) are diagrams illustrating one example of tasks presented, to a subject to be evaluated, by a task presenter included in the cognitive function evaluation system according to the embodiment of the present invention.
[FIG. 4]
   A diagram illustrating another example of cognitive tasks presented, to a subject to be evaluated, by the task presenter included in the cognitive function evaluation system according to the embodiment of the present invention.
[FIG. 5]
   Part (a) is a diagram illustrating a skeleton generated by a skeleton generator included in the cognitive function evaluation system according to the embodiment of the present invention. Part (b) is a diagram illustrating normalization processing performed by the skeleton generator.
[FIG. 6]
   A diagram illustrating one example of time-series data indicating motion of a joint corresponding to the right knee of a subject to be evaluated who is stepping in place and one example of a plurality of period-based joint data items whose phases are aligned.
[FIG. 7]
   A diagram illustrating one example of stacked pairs of absolute correlation matrices and a sliding window.
[FIG. 8]
   A diagram illustrating a configuration of an evaluator included in the cognitive function evaluation system according to the embodiment of the present invention.
[FIG. 9]
   A diagram illustrating a neural network of a motion feature extraction device included in the cognitive function evaluation system according to the embodiment of the present invention.
[FIG. 10]
   A schematic diagram illustrating spatial convolution processing, phase convolution processing, and period convolution processing.
[FIG. 11]
   A diagram illustrating a neural network of a first graph convolutional block included in the cognitive function evaluation system according to the embodiment of the present invention.
[FIG. 12]
   A diagram illustrating a neural network of an answer feature extraction device included in the cognitive function evaluation system according to the embodiment of the present invention.
[FIG. 13]
   A diagram illustrating a neural network of a fusion device included in the cognitive function evaluation system according to the embodiment of the present invention.

### [Description of Embodiments]

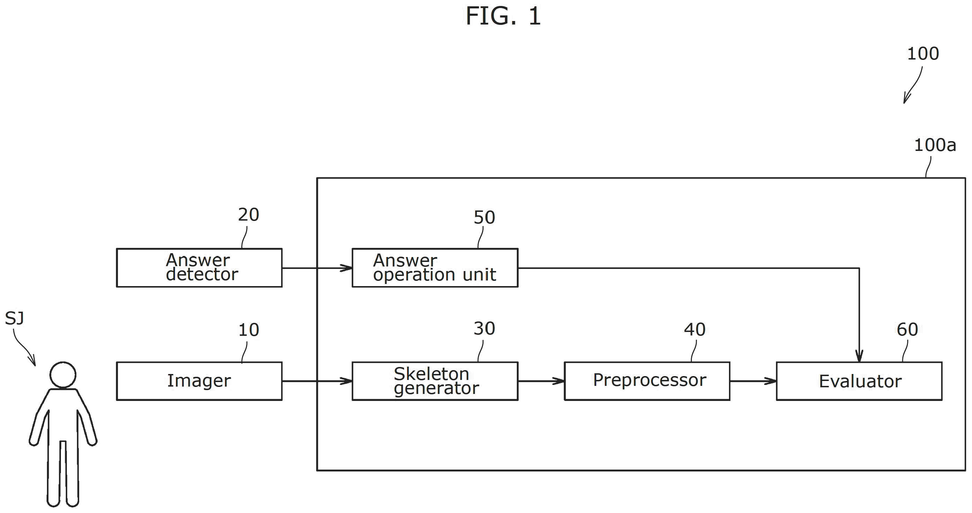

Hereinafter, an embodiment according to a cognitive function evaluation system of the present invention will be described with reference to the drawings (FIG. 1 through FIG. 13). However, the present invention is not limited to the following embodiment, and can be implemented in various aspects without departing from the scope of the gist of the present invention. Note that redundant description may be omitted as appropriate. Moreover, the same reference sign is given to the same or equivalent element throughout the drawings to avoid repetitive descriptions.

FIG. 1 is a diagram illustrating cognitive function evaluation system 100 according to the present embodiment. Cognitive function evaluation system 100 evaluates a cognitive function of subject SJ to be evaluated. To go into more detail, cognitive function evaluation system 100 of the present embodiment classifies the cognitive function of subject SJ to be evaluated into (i) a class of dementia or cognitive impairment and (ii) a class including mild cognitive impairment (MCI) and non-dementia. Alternatively, cognitive function evaluation system 100 classifies the cognitive function of subject SJ to be evaluated into (i) a class including dementia or cognitive impairment and mild cognitive impairment and (ii) a class of non-dementia. Hereinafter, dementia or cognitive impairment may be referred to as "dementia".

Note that mild cognitive impairment is of the stage prior to dementia, and indicates an intermediate state between a normal state and a dementia state. Specifically, mild cognitive impairment indicates a state in which a decline in cognitive functions such as memory and attention is observed, but not to an extent that interfere with daily life.

As shown in FIG. 1, cognitive function evaluation system 100 includes imager 10, answer detector 20, and information processor 100a.

Imager 10 images subject SJ to be evaluated performing a dual-task, and generates an imaging signal. The dual-task is a task in which a cognitive task and a motor task are simultaneously assigned to subject SJ to be evaluated. The cognitive task requires subject SJ to be evaluated to provide an answer to a cognitive test item. The cognitive test item includes, for example, a calculation test item, a spatial memory test item, or a rock-paper-scissors test item. The motor task includes, for example, stepping in place, walking, running, or skipping. The imaging signal is used for extraction of a motion feature of subject SJ to be evaluated.

Specifically, imager 10 images subject SJ to be evaluated, and generates a captured image. For example, imager 10 generates moving image data or video data. Imager 10 may include, for example, a charge-coupled device (CCD) image sensor or a complementary metal oxide semiconductor (CMOS) image sensor. For example, as imager 10, a video camera or an RGB camera may be used. Alternatively, as imager 10, a smartphone video camera may be used.

Imager 10 is provided, for example, in front of subject SJ to be evaluated. By providing imager 10 in front of subject SJ to be evaluated, a motion feature of subject SJ to be evaluated can be more reliably extracted.

In the present embodiment, imager 10 further images subject SJ to be evaluated performing a motor task (a single task). The motor task is a task in which a motor task is assigned to subject SJ to be evaluated.

In detail, in the present embodiment, subject SJ to be evaluated sequentially performs a cognitive task (a single task), a motor task (a single task), and a dual-task in the stated order. The cognitive task is a task that requires subject SJ to be evaluated to provide an answer to a cognitive task. Imager 10 images subject SJ to be evaluated while subject SJ to be evaluated is performing a motor task. Moreover, imager 10 images subject SJ to be evaluated while subject SJ to be evaluated is performing a dual-task. Hereinafter, a set of tasks to be performed by subject SJ to be evaluated may be referred to as a "task set".

More specifically, subject SJ to be evaluated performs a cognitive task for a predetermined time period (e.g., 30 seconds). Following the cognitive task, subject SJ to be evaluated performs a motor task for a predetermined time period (e.g., 20 seconds). Then, following the motor task, subject SJ to be evaluated performs a dual-task for a predetermined time period (e.g., 30 seconds). Hereinafter, the time period during which subject SJ to be evaluated performs a cognitive task may be referred to as a "cognitive task performance time". Likewise, the time period during which subject SJ to be evaluated performs a motor task may be referred to as a "motor task performance time" and the time period during which subject SJ to be evaluated performs a dual-task may be referred to as a "dual-task performance time". The length of a cognitive task performance time may be set (determined) as appropriate. Likewise, the length of a motor task performance time and the length of a dual-task performance time may be set (determined) as appropriate.

Note that the motor task of the motor task and the motor task of the dual-task may be the same motor task or different motor tasks. Likewise, the cognitive task of the cognitive task and the cognitive task of the dual-task may be the same cognitive task or different cognitive tasks. In the present embodiment, the motor task of the motor task and the motor task of the dual-task are the same motor task. Likewise, the cognitive task of the cognitive task and the cognitive task of the dual-task are the same cognitive task.

Answer detector 20 detects the answer given by subject SJ to be evaluated to a cognitive task, and generates a detection signal. In the present embodiment, answer detector 20 detects the answer given by subject SJ to be evaluated performing a cognitive task and the answer given by subject SJ to be evaluated performing a dual-task. Answer detector 20 may include, for example, a left-hand answer switch (not illustrated) and a right-hand answer switch (not illustrated). The detection signal is used for extraction of an answer feature of subject SJ to be evaluated.

An imaging signal from imager 10 is input to information processor 100a. Information processor 100a processes the imaging signal to extract a motion feature of subject SJ to be evaluated, and evaluates a cognitive function of subject SJ to be evaluated, based on the extracted motion feature of subject SJ to be evaluated. Information processor 100a may be implemented by a computer like, for example, a personal computer.

In the present embodiment, a detection signal from answer detector 20 is also input to information processor 100a. Information processor 100a processes the detection signal to extract an answer feature of the answer given by subject SJ to be evaluated to a cognitive task. Thereafter, information processor 100a evaluates the cognitive function of subject SJ to be evaluated, based on the motion feature of subject SJ to be evaluated and the answer feature of the answer given by subject SJ to be evaluated to the cognitive task.

More specifically, information processor 100a functions as skeleton generator 30, preprocessor 40, answer operation unit 50, and evaluator 60.

Skeleton generator 30 generates, based on imaging signals generated by imager 10, time-series data of skeleton SK representing subject SJ to be evaluated performing a dual-task. Stated differently, skeleton generator 30 generates a skeleton sequence representing subject SJ to be evaluated performing the dual-task. The time-series data of skeleton SK (a skeleton sequence) represents motion of subject SJ to be evaluated. Skeleton generator 30 is one example of the "generator".

In the present embodiment, skeleton generator 30 generates a skeleton sequence (time-series data of skeleton SK) that represents motion of subject SJ to be evaluated after performance of a cognitive task (motion of subject SJ to be evaluated sequentially performing a motor task and a dual-task in the stated order).

Preprocessor 40 detects periods of the time-series data of skeleton SK. Thereafter, preprocessor 40 decomposes the time-series data of skeleton SK into periods to generate a plurality of period-based skeleton data SKD items, and aligns phases of each period-based skeleton data SKD item to generate first input data. Accordingly, the first input data indicates the plurality of period-based skeleton data SKD items whose phases are aligned. Each period-based skeleton data SKD item indicates a plurality of skeletons SK arranged along the phase dimension. More specifically, each period-based skeleton data SKD item indicates skeleton SK for each specific phase point.

Stated differently, preprocessor 40 decomposes a skeleton sequence into periods to generate a plurality of period-based skeleton sequences, and aligns the phases of each period-based skeleton sequence to generate the first input data. Accordingly, the first input data indicates a plurality of period-based skeleton data sequences whose phases are aligned. Each period-based skeleton sequence indicates motion of skeleton SK along the phase dimension.

In detail, the motion of subject SJ to be evaluated performing a motor task has periodicity. For example, when a motor task is any one of stepping in place, walking, running, or skipping, the motion of subject SJ to be evaluated performing the motor task is quasi-periodic. Likewise, the motion of subject SJ to be evaluated performing a dual-task has periodicity. Therefore, preprocessor 40 can detect periods of the time-series data of skeleton SK.

Answer operation unit 50 generates second input data based on a detection signal generated by answer detector 20. The second input data indicates, for example, an answer speed and a correct answer rate. The answer speed indicates the number of times subject SJ to be evaluated answered per unit time. The unit time is, for example, one second. The correct answer rate indicates a ratio between the number of answers and the number of correct answers. The number of answers indicates the number of times subject SJ to be evaluated answered. The number of correct answers indicates the number of answers that are correct.

In the present embodiment, the answer speed includes a first answer speed and a second answer speed. The first answer speed is calculated by dividing the number of times subject SJ to be evaluated answered during performance of the cognitive task by the cognitive task performance time. The second answer speed is calculated by dividing the number of times subject SJ to be evaluated answered during performance of the dual-task by the dual-task performance time.

Likewise, the correct answer rate includes a first correct answer rate and a second correct answer rate. The first correct answer rate is a ratio between the number of times subject SJ to be evaluated answered during performance of the cognitive task and the number of times subject SJ to be evaluated correctly answered to test items presented during performance of the cognitive task. The second correct answer rate is a ratio between the number of times subject SJ to be evaluated answered during performance of the dual-task and the number of times subject SJ to be evaluated correctly answered to test items presented during performance of the dual-task.

Evaluator 60 extracts a motion feature of subject SJ to be evaluated, based on data (first input data) input from preprocessor 40. Thereafter, evaluator 60 evaluates a cognitive function of subject SJ to be evaluated, based on the extracted feature.

In the present embodiment, evaluator 60 extracts, based on the first input data, a feature of motion of subject SJ to be evaluated after subject SJ to be evaluated performed the cognitive task (the motion of subject SJ to be evaluated sequentially performing the motor task and the dual-task in the stated order). In addition, evaluator 60 further extracts, based on data (second input data) input from answer operation unit 50, an answer feature of the answer given by subject SJ to be evaluated to the cognitive task. Thereafter, evaluator 60 evaluates the cognitive function of subject SJ to be evaluated, based on the motion feature of subject SJ to be evaluated and the answer feature of subject SJ to be evaluated.

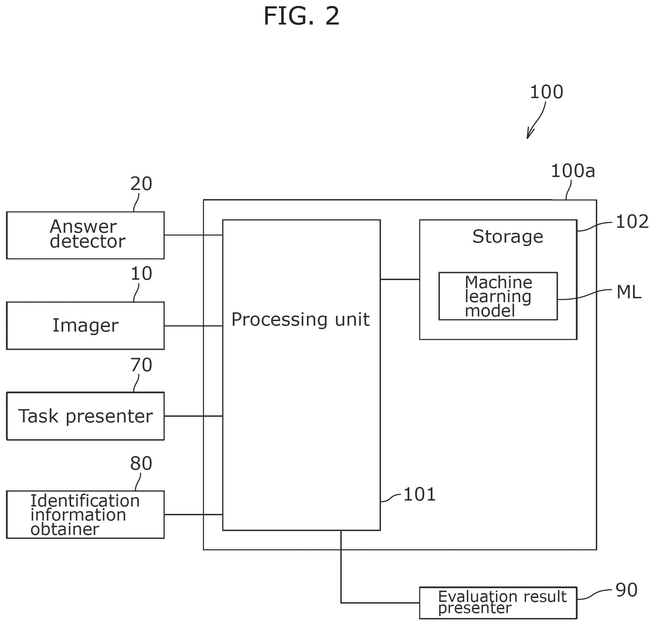

Next, cognitive function evaluation system 100 according to the present embodiment will be described with reference to FIG. 2. FIG. 2 is a block diagram illustrating a hardware configuration of cognitive function evaluation system 100 according to the present embodiment. As shown in FIG. 2, cognitive function evaluation system 100 further includes task presenter 70, identification information obtainer 80, and evaluation result presenter 90. Moreover, information processor 100a includes processing unit 101 and storage 102.

Task presenter 70 presents a task to be performed by subject SJ to be evaluated. In the present embodiment, task presenter 70 includes a display like, for example, a liquid crystal display or an organic electro-luminescence (EL) display. Task presenter 70 causes the display to display a screen showing a task to be performed by subject SJ to be evaluated. The display is provided, for example, in front of subject SJ to be evaluated.

Each of subjects SJ to be evaluated who use cognitive function evaluation system 100 is preassigned unique identification information. Identification information obtainer 80 obtains the identification information assigned to each subject SJ to be evaluated. Identification information obtainer 80 includes, for example, a card reader, a keyboard, or a touch panel. When identification information obtainer 80 includes a card reader, each subject SJ to be evaluated causes the card reader to read the identification information carried by a card. When identification information obtainer 80 includes a keyboard or a touch panel, each subject SJ to be evaluated operates the keyboard or the touch panel to input the assigned identification information.

Storage 102 stores various types of computer programs and various types of data items. Specifically, storage 102 includes a memory device. The memory device includes, for example, a semiconductor memory (semiconductor memory device). As the semiconductor memory, storage 102 may include, for example, read-only memory (ROM) and random-access memory (RAM). Storage 102 may further include, as the semiconductor memory, video RAM (VRAM). In addition, storage 102 may include a storage device. As the storage device, storage 102 may include, for example, at least one of a hard disk drive (HDD) or a solid state drive (SSD). Storage 102 may further include a removable medium.

In the present embodiment, storage 102 stores machine learning model ML. Machine learning model ML is a computer program that functions as evaluator 60 described with reference to FIG. 1, and outputs evaluation data indicating an evaluation result of the cognitive function of subject SJ to be evaluated, based on data (first input data) output from preprocessor 40 (see FIG. 1). In the present embodiment, machine learning model ML outputs evaluation data based on the data (first input data) output from preprocessor 40 (see FIG. 1) and the data (second input data) output from answer operation unit 50 (see FIG. 1).

Note that first input data items and second input data items collected by causing a plurality of subjects to perform task sets are used as explanatory variables in a plurality of training data items to be learned by machine learning model ML. Moreover, label data items (correct answer labels) given to the plurality of subjects are used as target variables in the plurality of training data items.

Specifically, when machine learning model ML is generated which classifies the cognitive function of subject SJ to be evaluated into a class of dementia and a class including mild cognitive impairment and non-dementia, the label data indicates whether the subject is in the class of dementia (positive) or in the class including mild cognitive impairment and non-dementia (negative). Likewise, when machine learning model ML is generated which classifies the cognitive function of subject SJ to be evaluated into a class including dementia and mild cognitive impairment and a class of non-dementia, the label data indicates whether the subject is in the class including dementia and mild cognitive impairment (positive) or in the class of non-dementia (negative). For example, the label data may be generated based on the result of definitive diagnosis by a doctor.

Processing unit 101 executes the various types of computer programs stored in storage 102 to perform various processes such as numerical calculations, information processing, and device control. Specifically, processing unit 101 includes a processor. The processor may include, for example, a central processing unit (CPU), a graphics processing unit (GPU), or a neural network processing unit (NPU). Alternatively, processing unit 101 may include a quantum computer.

In detail, processing unit 101 functions as skeleton generator 30, preprocessor 40, answer operation unit 50, and evaluator 60 shown in FIG. 1 by executing the various types of computer programs stored in storage 102.

Specifically, when identification information is obtained by identification information obtainer 80, processing unit 101 controls task presenter 70 to present a task to be performed by subject SJ to be evaluated. Thereafter, processing unit 101 obtains an evaluation result of the cognitive function of subject SJ to be evaluated, based on an imaging signal output from imager 10 and a detection signal output from answer detector 20.

In the present embodiment, the evaluation result includes a classification result of the cognitive function. The classification result indicates a class into which the cognitive function of subject SJ to be evaluated is classified. Specifically, classes into which the cognitive function of subject SJ to be evaluated is classified include two classes. One of the two classes indicates the class of dementia. The other of the two classes indicates the class including mild cognitive impairment and non-dementia. Alternatively, one of the two classes indicates the class including dementia and mild cognitive impairment. The other of the two classes indicates the class of non-dementia.

When processing unit 101 obtains the evaluation result of the cognitive function of subject SJ to be evaluated, processing unit 101 causes storage 102 to store the evaluation result of the cognitive function of subject SJ to be evaluated in association with the identification information assigned to subject SJ to be evaluated. As a result, the history of evaluation results of the cognitive function is stored in storage 102 in association with the identification information.

Under control of processing unit 101, evaluation result presenter 90 presents, to subject SJ to be evaluated, an evaluation result of the cognitive function or the history of evaluation results of the cognitive function. Evaluation result presenter 90 includes, for example, a printer. Alternatively, evaluation result presenter 90 may include a communication device.

When cognitive function evaluation system 100 includes a printer as evaluation result presenter 90, processing unit 101 controls the printer to cause the printer to output a sheet on which the evaluation result of the cognitive function is printed. Alternatively, processing unit 101 generates a table or a graph showing the history of evaluation results of the cognitive function, and causes the printer to output a sheet on which the table or the graph showing the history of evaluation results of the cognitive function is printed. When cognitive function evaluation system 100 includes a communication device as evaluation result presenter 90, processing unit 101 may control the communication device to transmit mail indicating the evaluation result of the cognitive function of subject SJ to be evaluated to, for example, a mail address registered in advance by subject SJ to be evaluated. Processing unit 101 may alternatively transmit mail indicating a table or a graph showing the history of evaluation results of the cognitive function.

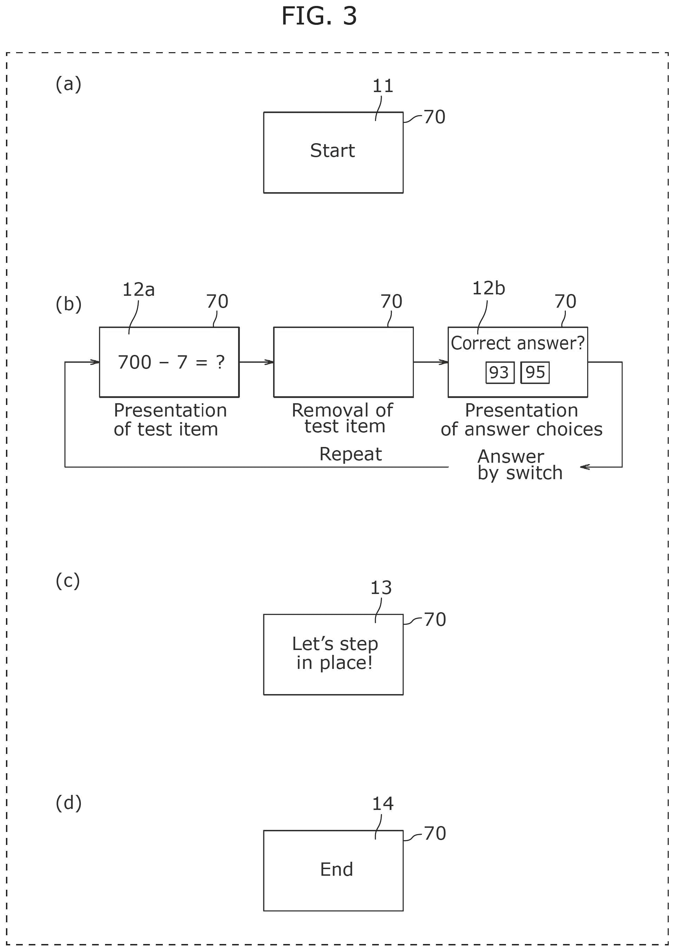

Next, one example of a task presented by task presenter 70 to subject SJ to be evaluated will be described with reference to part (a) of FIG. 3 through part (d) of FIG. 3. Part (a) of FIG. 3 through part (d) of FIG. 3 show one example of a task presented, to subject SJ to be evaluated, by task presenter 70 included in cognitive function evaluation system 100 according to the present embodiment. In the example shown in part (a) of FIG. 3 through part (d) of FIG. 3, a motor task assigned to subject SJ to be evaluated is "stepping in place" and a cognitive task (cognitive test item) assigned to subject SJ to be evaluated is a "calculation test item".

As shown in part (a) of FIG. 3, under control of processing unit 101, task presenter 70 displays first notification screen 11 on the display to notify the start of a task to subject SJ to be evaluated. Next, as shown in part (b) of FIG. 3, under control of processing unit 101, task presenter 70 presents a cognitive task. Specifically, task presenter 70 displays test-item presentation screen 12a showing a calculation test item (a cognitive task) on the display. For example, task presenter 70 displays, as the calculation test item, a subtraction test item on the display.

Note that in the example shown in part (b) of FIG. 3, the calculation test item is a subtraction test item, but the calculation test item is not limited thereto. The calculation test item may be an addition test item. Or, the calculation test item may include a subtraction test item and an addition test item.

Under the control of processing unit 101, task presenter 70 ends display of the cognitive task (the calculation test item) at a predetermined timing. Specifically, test-item presentation screen 12a is removed from the display. After test-item presentation screen 12a is removed from the display, under control of processing unit 101, task presenter 70 displays answer-choice presentation screen 12b showing two answer choices on the display. Subject SJ to be evaluated presses one of two answer switches included in answer detector 20 to select the answer.

When subject SJ to be evaluated selects the answer, under control of processing unit 101, task presenter 70 displays the next calculation test item (the next cognitive task) on the display. In detail, as the next calculation test item, a calculation test item different from the previous calculation test item is displayed on the display. From this point onward, presentation of calculation test items (cognitive tasks) to be answered by subject SJ to be evaluated is repeated until a predetermined cognitive task performance time elapses.

As shown in part (c) of FIG. 3, after the predetermined cognitive task performance time has elapsed, task presenter 70 presents a motor task to subject SJ to be evaluated. Specifically, under control of processing unit 101, task presenter 70 displays, on the display, motor presentation screen 13 showing a motor task (stepping in place) assigned to subject SJ to be evaluated.

In the present embodiment, the motor task assigned to subject SJ to be evaluated in the dual-task is the same as the motor task assigned to subject SJ to be evaluated in the motor task. Moreover, the cognitive task assigned to subject SJ to be evaluated in the dual-task is the same as the cognitive task assigned to subject SJ to be evaluated in the cognitive task. Accordingly, after the predetermined motor task performance time has elapsed, task presenter 70 repeats the presentation of calculation test items (cognitive tasks) to be answered by subject SJ to be evaluated until a predetermined dual-task performance time elapses, as described with reference to part (b) of FIG. 3.

As shown in part (d) of FIG. 3, after the predetermined dual-task performance time has elapsed, under control of processing unit 101, task presenter 70 displays, on the display, second notification screen 14 to notify the end of the task to subject SJ to be evaluated.

Note that the cognitive task presented by task presenter 70 is not limited to a calculation test item. For example, the cognitive task may be a spatial memory test item or a rock-paper-scissors test item.

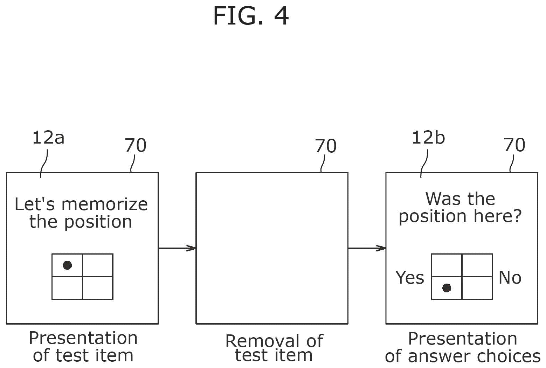

FIG. 4 is a diagram illustrating another example of cognitive tasks presented to subject SJ to be evaluated by task presenter 70 included in cognitive function evaluation system 100 according to the present embodiment. In the example shown in FIG. 4, the cognitive task is a "spatial memory test item". As shown in FIG. 4, when the cognitive task is a spatial memory test item, under control of processing unit 101, task presenter 70 displays, on the display, test-item presentation screen 12a showing a figure placed in one of four areas in which the figure can be placed.

After test-item presentation screen 12a is removed from the display, under control of processing unit 101, task presenter 70 displays answer-choice presentation screen 12b showing the figure placed in one of the four areas in which the figure can be placed. On answer-choice presentation screen 12b, "Yes" and "No" are displayed as the two answer choices together with a test item text. The test item text indicates a test item that can be answered by either "Yes" or "No". Here, subject SJ to be evaluated is asked whether the position in which the figure was placed is the same on test-item presentation screen 12a and answer-choice presentation screen 12b.

When subject SJ to be evaluated determines that the position in which the figure was placed is the same on test-item presentation screen 12a and answer-choice presentation screen 12b, subject SJ to be evaluated selects "Yes" by pressing the left-hand answer switch. Whereas when subject SJ to be evaluated determines that the position in which the figure was placed is different on test-item presentation screen 12a and answer-choice presentation screen 12b, subject SJ to be evaluated selects "No" by pressing the right-hand answer switch.

Next, the "rock-paper-scissors test item" will be described. When the cognitive task assigned to subject SJ to be evaluated is the "rock-paper-scissors test item", for example, task presenter 70 displays one of "rock", "scissors", or "paper" on test-item presentation screen 12a. Thereafter, on answer-choice presentation screen 12b, one of "rock", "scissors", or "paper", a test item text, and "Yes" and "No" are displayed. On answer-choice presentation screen 12b, subject SJ to be evaluated is asked, for example, whether the finger pose displayed on answer-choice presentation screen 12b can beat the finger pose previously displayed on test-item presentation screen 12a.

When subject SJ to be evaluated determines that the finger pose displayed on answer-choice presentation screen 12b can beat the finger pose previously displayed on test-item presentation screen 12a, subject SJ to be evaluated selects "Yes" by pressing the left-hand answer switch. Whereas, when subject SJ to be evaluated determines that the finger pose displayed on answer-choice presentation screen 12b cannot beat the finger pose previously displayed on test-item presentation screen 12a, subject SJ to be evaluated selects "No" by pressing the right-hand answer switch.

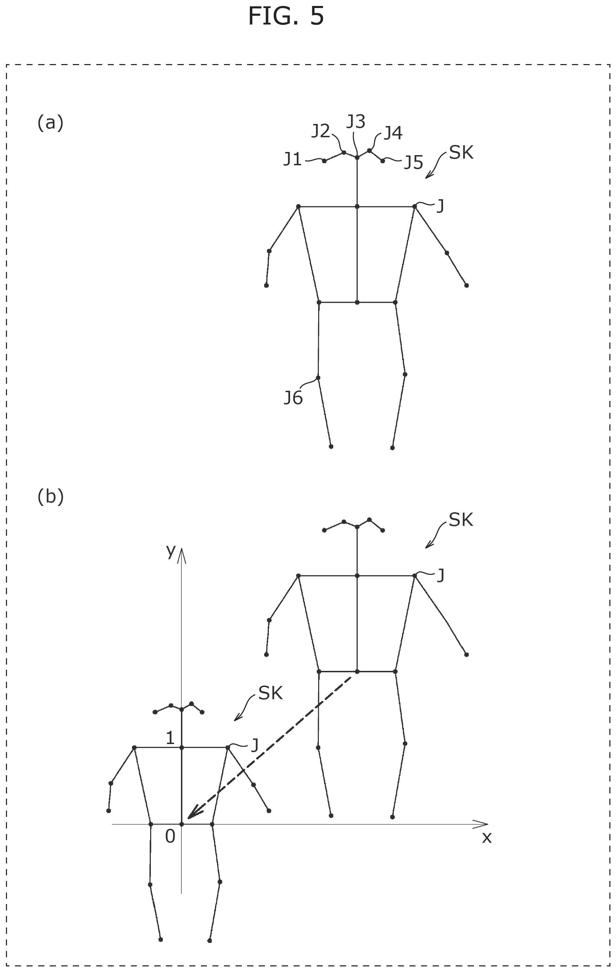

Next, skeleton generator 30 will be described with reference to FIG. 1 and part (a) of FIG. 5. Part (a) of FIG. 5 is a diagram illustrating skeleton SK that is generated by skeleton generator 30 included in cognitive function evaluation system 100 according to the present embodiment. As has been already described, skeleton generator 30 generates time-series data (a skeleton sequence) of skeleton SK, based on an imaging signal generated by imager 10.

For example, skeleton generator 30 (processing unit 101) may process each of the frames included in moving image data or video data using a machine learning model to generate skeleton SK for each frame. An algorithm used by the machine learning model may be, for example, real-time multi-person pose estimation (RTMpose). To go into more detail, skeleton generator 30 may resample the moving image data or the video data into 10 frames per second to generate skeleton SK for each resampled frame.

As shown in part (a) of FIG. 5, in the present embodiment, skeleton generator 30 generates two-dimensional skeleton SK for each frame. Skeleton SK shows a graph structure including a plurality of joints J. Accordingly, the time-series data (the skeleton sequence) of skeleton SK indicates the time-series data of each of the plurality of joints J constituting skeleton SK.

In the present embodiment, skeleton SK represents the whole body of subject SJ to be evaluated. Skeleton SK shown in part (a) of FIG. 5 includes 17 joints J. Note that joints J constituting skeleton SK need not correspond to the joints in the human body. For example, skeleton SK shown in part (a) of FIG. 5 includes first joint J1, second joint J2, third joint J3, fourth joint J4, and fifth joint J5. First joint J1 corresponds to the right ear of subject SJ to be evaluated. Second joint J2 corresponds to the right eye of subject SJ to be evaluated. Third joint J3 corresponds to the nose of subject SJ to be evaluated. Fourth joint J4 corresponds to the left eye of subject SJ to be evaluated. Fifth joint J5 corresponds to the left ear of subject SJ to be evaluated.

Next, skeleton generator 30 will be described with reference to FIG. 1 and part (b) of FIG. 5. Part (b) of FIG. 5 illustrates normalization processing performed by skeleton generator 30. In the present embodiment, skeleton generator 30 normalizes skeleton SK of each frame, and generates the time-series data (skeleton sequence) of skeleton SK. Normalization of skeleton SK can prevent individual physique differences from affecting the evaluation result of the cognitive function. Normalization of skeleton SK can also prevent the location in which subject SJ to be evaluated performs tasks from affecting the evaluation result of the cognitive function.

Specifically, as shown in part (b) of FIG. 5, skeleton generator 30 scales skeleton SK such that the length of the spine is equal to a unit length. Moreover, skeleton generator 30 moves skeleton SK such that the hip joint of skeleton SK is positioned at the origin point of the xy coordinate system. Here, the length of the spine indicates the length between the hip joint and the neck joint. Note that the hip joint and the neck joint need not be parts of joints J (here, 17 joints J) of skeleton SK. For example, skeleton generator 30 may calculate the position of the neck joint based on the mean position of the right and the left shoulder joints. Moreover, skeleton generator 30 may calculate the position of the hip joint based on the mean position of the pelvis.

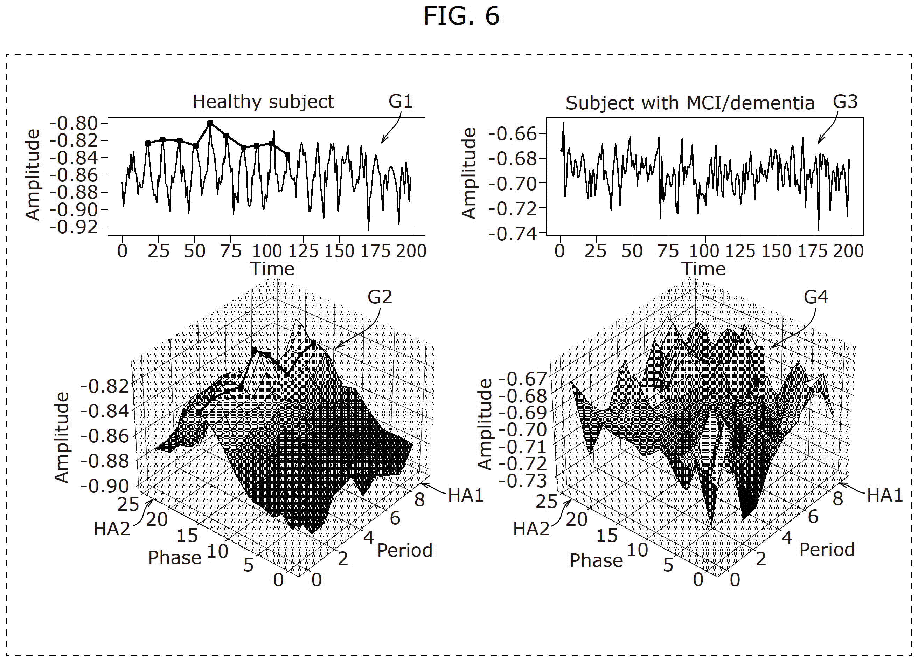

Next, preprocessor 40 will be described with reference to FIG. 1 and FIG. 6. In the present embodiment, preprocessor 40 detects periods of the time-series data of each joint J. Thereafter, preprocessor 40 decomposes the time-series data of each joint J into the periods, and, for each joint J, generates a plurality of period-based joint data items. Thereafter, for each joint J, preprocessor 40 aligns phases of the plurality of period-based joint data items to generate first input data. Accordingly, the first input data indicates, for each joint J, a plurality of period-based joint data items whose phases are aligned. Each period-based joint data item indicates motion along the phase dimension of a corresponding joint J.

For example, preprocessor 40 may decompose the time-series data of each of joints J constituting skeleton SK into periods to generate a plurality of period-based joint data items for each joint J. The plurality of period-based joint data items indicate motion of joint J along the phase dimension for each period. Note that, in the following description, a plurality of period-based joint data items whose phases are aligned will be referred to as a "period-based joint dataset". The first input data indicates the period-based joint dataset of each joint J.

In detail, the time-series data of each of a plurality of joints J constituting two-dimensional skeleton SK includes the time-series data indicating motion of joint J in the x direction and the time-series data indicating motion of joint J in the y direction. For each joint J, preprocessor 40 decomposes the time-series data (one-dimensional time-series data) indicating motion of joint J in the x direction into periods, and generates period-based first joint data items of joint J. The period-based first joint data item indicates a first joint data item of each period. Likewise, for each joint J, preprocessor 40 decomposes the time-series data (one-dimensional time-series data) indicating motion of joint J in the y direction into periods, and generates period-based second joint data items of joint J. The period-based second joint data item indicates a second joint data item of each period. Thereafter, for each joint J, preprocessor 40 aligns phases of the plurality of first joint data items and phases of the plurality of second joint data items.

For example, preprocessor 40 (processing unit 101) may decompose one-dimensional time-series data into periods based on a self-dynamic time warping (Self-DTW) algorithm and may obtain samples corresponding to the same phase from respective decomposed periods to align the phases of the plurality of period-based joint data items. Stated differently, preprocessor 40 may sample data items of fixed number Nₚₕₐₛₑ of phase points from each decomposed period to align the phases of the plurality of period-based joint data items. By sampling the data items of fixed number Nₚₕₐₛₑ of phase points, the number of phases included in each period-based joint data item can be made to equal fixed number Nₚₕₐₛₑ. To go into more detail, based on the Self-DTW algorithm, preprocessor 40 (processing unit 101) may convert one-dimensional time-series data into an "N × M" matrix. Here, "N" denotes the number of periods, and "M" denotes time-series points of each period after phase alignment.

Note that in the following description, a period-based first joint data item may be referred to as a "first period-based joint data item". Likewise, a period-based second joint data item may be referred to as a "second period-based joint data item". Moreover, period-based first joint data items whose phases are aligned may be referred to as a "first period-based joint dataset". Likewise, period-based second joint data items whose phases are aligned may be referred to as a "second period-based joint dataset". The period-based joint dataset of each joint J indicates the first period-based joint dataset and the second period-based joint dataset.

FIG. 6 is a diagram illustrating one example of time-series data indicating motion of joint J6 (see part (a) of FIG. 5) corresponding to the right knee of subject SJ to be evaluated who is stepping in place and one example of a plurality of period-based joint data items (a period-based joint dataset) whose phases are aligned.

In detail, time-series data G1 in the upper-left of FIG. 6 shows the y-direction motion of joint J6 (see part (a) of FIG. 5) corresponding to the right knee of a healthy subject (subject SJ to be evaluated without dementia). Period-based joint dataset G2 in the lower-left of FIG. 6 shows a plurality of period-based joint data items (a second period-based joint dataset) obtained by decomposing time-series data G1 into periods and aligning the phases. Time-series data G3 in the upper-right of FIG. 6 shows the y-direction motion of joint J6 (see part (a) of FIG. 5) corresponding to the right knee of subject SJ to be evaluated with dementia. Period-based joint dataset G4 in the lower-right of FIG. 6 shows a plurality of period-based joint data items (a second period-based joint dataset) obtained by decomposing time-series data G3 into periods and aligning the phases.

Moreover, in the upper-right and upper-left diagrams in FIG. 6, the horizontal axis represents time, and the vertical axis represents the amplitude of joint J6 in the y direction. In the lower-right and lower-left diagrams in FIG. 6, first horizontal axis HA1 represents period and second horizontal axis HA2 represents phase, and the vertical axis represents the amplitude of joint J6 in the y direction. In detail, the vertical axis represents the y-coordinate of the xy coordinate system (see part (b) of FIG. 5) whose origin is at the hip joint.

Note that when the time-series data indicating the y-direction motion of joint J6 corresponding to the right knee of subject SJ to be evaluated performing stepping in place is decomposed into periods, period-based joint data items are generated, with two steps of stepping in place being treated as one period.

As shown in FIG. 6, the motion of the healthy subject is periodic, whereas the motion of subject SJ to be evaluated with dementia is non-periodic. Accordingly, the use of period-based skeleton sequences (period-based joint data items) enables the cognitive function of subject SJ to be evaluated to be more accurately evaluated.

In addition, the use of period-based skeleton sequences (period-based joint data items) whose phases are aligned enables analysis of variation in samples between different periods corresponding to the same phase. Accordingly, the cognitive function of subject SJ to be evaluated can be more accurately evaluated.

Furthermore, according to the present embodiment, samples of different periods corresponding to the same phase are placed in close proximity within a three-dimensional space. Accordingly, convolution can be performed using a smaller kernel size compared to convolution of one-dimensional time-series data. As a result, both long-term and short-term relationships between samples can be analyzed. Therefore, the cognitive function of subject SJ to be evaluated can be more accurately evaluated.

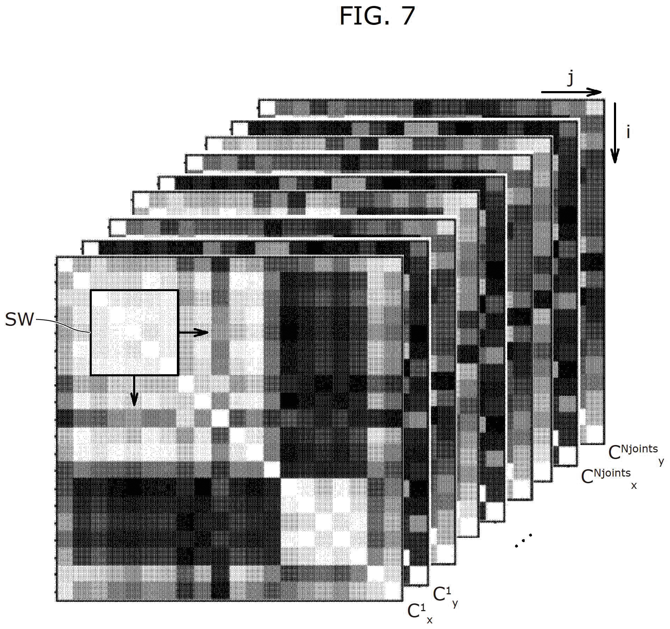

Next, preprocessor 40 will be described with reference to FIG. 1 and FIG. 7. In order to process data using machine learning model ML (neural network NW), the number of variables input to machine learning model ML needs to be adjusted to a fixed number. Accordingly, in order to process period-based skeleton data SKD items whose phases are aligned using machine learning model ML, the number of period-based skeleton data SKD items (the number of periods) needs to be adjusted to a fixed number. With this being the case, in the present embodiment, preprocessor 40 adjusts the number of period-based skeleton data SKD items (the number of periods) to a fixed number to generate first input data.

Specifically, preprocessor 40 selects N_{periods} (a fixed number) periods from among M_{periods} periods extracted from time-series data of each joint J to make the number of period-based joint data items (the number of periods) of each joint J equal the fixed number. For example, for each joint J, preprocessor 40 selects a fixed number of (N_{periods}) consecutive periods (period-based joint data items) having the highest correlation. Note that when the number of (M_{periods}) period-based joint data items (periods) extracted from one-dimensional time-series data is less than the fixed number (N_{periods}), preprocessor 40 repeatedly uses some or all of the extracted M_{periods} period-based joint data items to make the number of period-based joint data items match the fixed number (N_{periods}). For example, when period-based joint data items corresponding to seven periods {P1, P2, P3, P4, P5, P6, P7} are extracted under the condition of "N_{periods} = 10", preprocessor 40 incorporates period-based joint data items corresponding to the three periods {P1, P2, P3} out of the extracted seven periods into the seven period-based joint data items. As a result, the period-based joint data items corresponding to 10 periods {P1, P2, P3, P4, P5, P6, P7, P1, P2, P3} are input to machine learning model ML.

For example, preprocessor 40 may calculate, for each joint J, a pair of absolute correlation matrices of the x dimension and y dimension, based on the following expression (1). Note that, in expression (1), "M" denotes M_{periods}. ${C}^{jointx} , {C}^{jointy} ∈ \left[0, 1\right] M \times M$ (1)

In expression (1), "C^{jointx}" and "C^{jointy}" denote the Pearson correlation coefficients between the i-th and j-th periods extracted from the time-series data of joint J. More specifically, the Pearson correlation coefficient is defined by the following expression (2).
[Math. 1] ${C}_{i , j}^{joint} = \left|\frac{cov \left({P}_{i}^{joint}, {P}_{j}^{joint}\right)}{{σ}_{{P}_{i}^{joint}} {σ}_{{P}_{j}^{joint}}}\right|$ (2) [Math. 2] $cov \left({P}_{i}^{joint}, {P}_{j}^{joint}\right)$ (3) [Math. 3] ${σ}_{{P}_{i}^{joint}} {σ}_{{P}_{j}^{joint}}$ (4)

Note that expression (3) included in expression (2) shows the covariance between the i-th and J-th periods extracted from the time-series data of joint J, and expression (4) included in expression (2) shows the standard deviation between the i-th and J-th periods extracted from the time-series data of joint J.

Furthermore, in order to search for start index k of the fixed number of (N_{periods}) consecutive periods (period-based joint data items) having the highest correlation from the extracted M_{periods} periods, preprocessor 40 stacks pairs of absolute correlation matrices (C^{jointx}, C^{jointy}). Thereafter, preprocessor 40 searches for start index k from the stacked pairs of absolute correlation matrices using sliding window SW having the size of N_{periods} × N_{periods}. FIG. 7 is a diagram illustrating one example of stacked pairs of absolute correlation matrices (C^{jointx}, C^{jointy}) and sliding window SW. For example, based on the following expression (5), preprocessor 40 may search for start index k having the highest absolute correlation within sliding window SW.
[Math. 4] $k = {arg max}_{x} \left({\sum }_{i = x}^{x + {N}_{periods}} {\sum }_{j = y}^{y + {N}_{periods}} {\sum }_{joint = 1}^{{N}_{joints}} {C}_{i , j}^{joint}\right)$ (5)

In expression (5), "x" and "y" are elements of the set [1, M_{periods} - N_{periods}] shown in the following expressions (6) and (7). $x ∈ \left[1, {M}_{periods}-{N}_{periods}\right]$ (6) $y ∈ \left[1, {M}_{periods}-{N}_{periods}\right]$ (7)

When start index k is found, preprocessor 40 extracts N_{periods} (fixed number) consecutive periods {P(k), P(k + 1), ..., P(k + N_{periods} - 1)} starting from start index k. Thereafter, preprocessor 40 inputs period-based joint data items corresponding to periods {P(k), P(k + 1), ..., P(k + N_{periods} - 1)} to evaluator 60 (see FIG. 1).

As has been described above, for each joint J, preprocessor 40 decomposes one-dimensional time-series data into periods, selects a fixed number of (N_{periods}) period-based joint data items from among a plurality of period-based joint data items whose phases are aligned, and inputs, as first input data (a period-based joint dataset), the selected period-based joint data items to evaluator 60 (see FIG. 1).

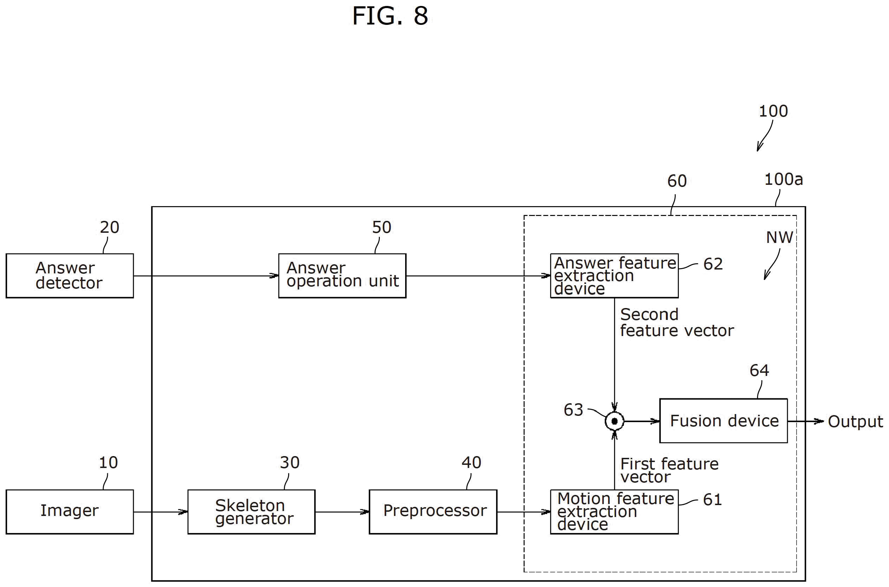

Next, cognitive function evaluation system 100 according to the present embodiment will be described with reference to FIG. 8. FIG. 8 is a diagram illustrating a configuration of evaluator 60 included in cognitive function evaluation system 100 according to the present embodiment. In detail, FIG. 8 illustrates neural network NW built by machine learning model ML shown in FIG. 2. Neural network NW is, for example, the neural network that performs deep learning.

As shown in FIG. 8, in the present embodiment, neural network NW includes motion feature extraction device 61, answer feature extraction device 62, coupling device 63, and fusion device 64.

Motion feature extraction device 61 extracts a motion feature of subject SJ to be evaluated performing a dual-task, based on first input data input from preprocessor 40. Thereafter, motion feature extraction device 61 outputs a first feature vector indicating the motion feature of subject SJ to be evaluated. In the present embodiment, motion feature extraction device 61 extracts a motion feature of motion of subject SJ to be evaluated after subject SJ to be evaluated performed the cognitive task (motion of subject SJ to be evaluated performing a motor task and a dual-task in the stated order).

In detail, motion feature extraction device 61 extracts the motion feature of subject SJ to be evaluated, based on a plurality of period-based skeleton data SKD items (period-based skeleton sequences) whose phases are aligned. In the present embodiment, motion feature extraction device 61 extracts the motion feature of subject SJ to be evaluated, based on a period-based joint dataset (a plurality of period-based joint data items whose phases are aligned) of each joint J. More specifically, motion feature extraction device 61 extracts the motion feature of subject SJ to be evaluated, based on a fixed number of (N_{periods}) first period-based joint data items and a fixed number of (N_{periods}) second period-based joint data items.

To go into more detail, the first period-based joint dataset of each joint J indicates phase-dimension data, period-dimension data, and spatial-dimension (x-dimension) data. Likewise, the second period-based joint dataset of each joint J indicates phase-dimension data, period-dimension data, and spatial-dimension (y-dimension) data. In other words, the first input data indicates the phase-dimension data items, the period-dimension data items, and the spatial-dimension data items. Accordingly, the number of dimensions of the first input data is three (spatial dimension, phase dimension, and period dimension). Motion feature extraction device 61 extracts the motion feature of subject SJ to be evaluated by convolving the first input data for each dimension (spatial dimension, phase dimension, and period dimension).

Note that the number of variables input to motion feature extraction device 61 from preprocessor 40 is "N_{dim} × N_{periods} × Nₚₕₐₛₑ × Nⱼₒᵢₙₜ". Here, "N_{dim}" denotes the dimension (spatial dimension) of skeleton SK. Since the present embodiment uses two-dimensional skeleton SK, "N_{dim} = 2" holds. As has been already described, "N_{periods}" denotes the number of periods, and "Nₚₕₐₛₑ" denotes the number of phases. In addition, "Nⱼₒᵢₙₜ" denotes the number of joints J. For example, when period-based joint datasets of all joints J of skeleton SK described with reference to part (a) of FIG. 5 are used, the number of joints J, i.e., Nⱼₒᵢₙₜ, is 17 (Nⱼₒᵢₙₜ = 17).

Answer feature extraction device 62 extracts an answer feature of subject SJ to be evaluated performing a dual-task, based on second input data input from answer operation unit 50. Thereafter, answer feature extraction device 62 outputs a second feature vector indicating the answer feature of subject SJ to be evaluated. In the present embodiment, answer feature extraction device 62 extracts an answer feature of the answer given by subject SJ to be evaluated to a cognitive task, based on the answer given by subject SJ to be evaluated performing a cognitive task and the answer given by subject SJ to be evaluated performing a dual-task.

In detail, answer feature extraction device 62 extracts the answer feature of subject SJ to be evaluated, based on the answer speed and the correct answer rate of subject SJ to be evaluated with respect to the cognitive task. More specifically, answer feature extraction device 62 extracts the answer feature of subject SJ to be evaluated, based on a first answer speed (the answer speed during performance of the cognitive task), a second answer speed (the answer speed during performance of the dual-task), a first correct answer rate (the correct answer rate during performance of the cognitive task), and a second correct answer rate (the correct answer rate during performance of the dual-task).

To go into more detail, answer feature extraction device 62 extracts the answer feature of subject SJ to be evaluated by convolving the second input data. Note that the number of variables input to answer feature extraction device 62 from answer operation unit 50 is four (the first answer speed, second answer speed, first correct answer rate, and second correct answer rate).

Coupling device 63 couples the first feature vector output from motion feature extraction device 61 and the second feature vector output from answer feature extraction device 62 to form a third feature vector. The third feature vector indicates a cross-modal feature vector. The third feature vector is input to fusion device 64.

Fusion device 64 fuses the first feature vector and the second feature vector, and outputs evaluation data (data showing an evaluation result of the cognitive function of subject SJ to be evaluated). In other words, fusion device 64 fuses the motion feature of subject SJ to be evaluated and the answer feature of subject SJ to be evaluated, and outputs the evaluation data. For example, the evaluation data shows a predicted probability of being positive. Being positive indicates, for example, that the cognitive function is classified into the class of dementia. Alternatively, being positive indicates that the cognitive function is classified into the class including dementia and mild cognitive impairment. In other words, as the evaluation data, fusion device 64 outputs data that classifies the cognitive function of subject SJ to be evaluated into the class of dementia (positive) and the class including mild cognitive impairment and non-dementia (negative). Alternatively, as the evaluation data, fusion device 64 outputs data that classifies the cognitive function of subject SJ to be evaluated into the class including dementia and mild cognitive impairment (positive) and the class of non-dementia (negative).

In the present embodiment, the first feature vector and the second feature vector are coupled together and input to fusion device 64. Because of this, the cognitive function of subject SJ to be evaluated can be evaluated without missing part of information indicating the motion feature of subject SJ to be evaluated. Likewise, the cognitive function of subject SJ to be evaluated can be evaluated without missing part of information indicating the answer feature of subject SJ to be evaluated. Accordingly, the cognitive function of subject SJ to be evaluated can be more accurately evaluated.

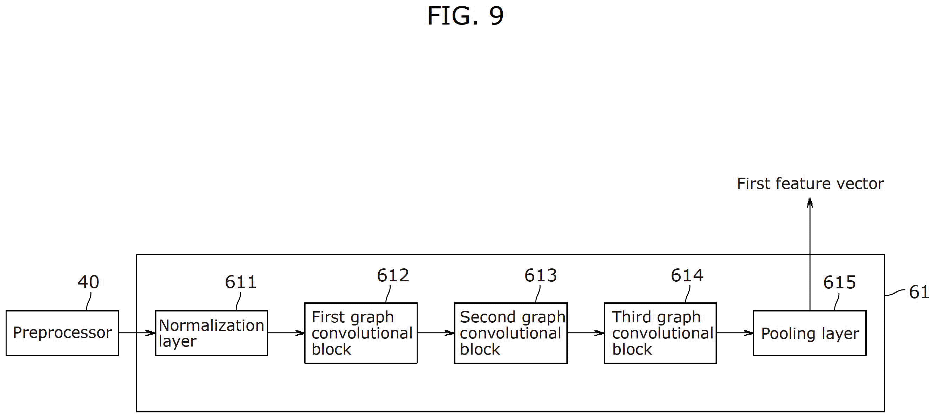

Next, one example of a network structure of motion feature extraction device 61 will be described with reference to FIG. 9. FIG. 9 is a diagram illustrating a neural network of motion feature extraction device 61 included in cognitive function evaluation system 100 according to the present embodiment.

Motion feature extraction device 61 shown in FIG. 9 includes normalization layer 611, first graph convolutional block 612, second graph convolutional block 613, third graph convolutional block 614, and pooling layer 615. Normalization layer 611, first graph convolutional block 612, second graph convolutional block 613, third graph convolutional block 614, and pooling layer 615 are connected in series in the stated order. Note that first graph convolutional block 612, second graph convolutional block 613, and third graph convolutional block 614 are one example of "a plurality of convolutional blocks".

The first input data (period-based joint dataset of each joint J) is input to normalization layer 611 from preprocessor 40. Normalization layer 611 normalizes the first input data. For example, normalization layer 611 may include a batch normalization layer. Since the number of dimensions of the first input data according to the present embodiment is three, normalization layer 611 may include three-dimensional batch normalization layer. The normalized first input data is input to first graph convolutional block 612.

First graph convolutional block 612 convolves each of the phase-dimension data, period-dimension data, and spatial-dimension data contained in the normalized first input data (period-based joint dataset of each joint J), and outputs a first feature map. The first feature map is input to second graph convolutional block 613.

Second graph convolutional block 613 and third graph convolutional block 614 have a configuration substantially the same as the configuration of first graph convolutional block 612. Second graph convolutional block 613 convolves each of the phase-dimension data, period-dimension data, and spatial-dimension data contained in the first feature map, and outputs a second feature map. Third graph convolutional block 614 convolves each of the phase-dimension data, period-dimension data, and spatial-dimension data contained in the second feature map, and outputs a third feature map.

Note that the number of channels of second graph convolutional block 613 may be less than the number of channels of first graph convolutional block 612. Note that the number of channels of third graph convolutional block 614 may be greater than the number of channels of second graph convolutional block 613. Note that the number of channels of third graph convolutional block 614 may be the same as the number of channels of first graph convolutional block 612.

In the present embodiment, a feature map (the third feature map) is input to pooling layer 615 from third graph convolutional block 614. Pooling layer 615 converts the feature map (the third feature map) into a first feature vector. Pooling layer 615 may include, for example, a global average pooling layer. Pooling layer 615 converts, for example, the feature map (the third feature map) into a feature vector (a first feature vector) having the vector length of 64.

According to the present embodiment, since the first input data is convolved using a plurality of convolutional blocks connected in series (here, three graph convolutional blocks), the number of operations performed in one convolutional block (one graph convolutional block) can be reduced.

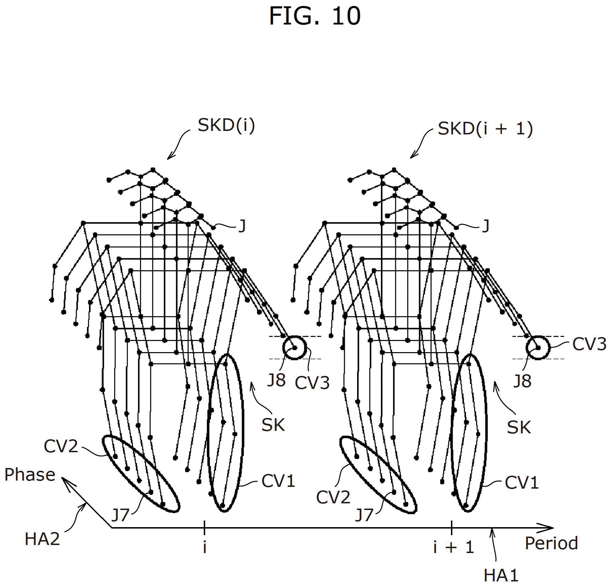

Next, with reference to FIG. 10, spatial convolution processing CV1 for convolving spatial-dimension data, phase convolution processing CV2 for convolving phase-dimension data, and period convolution processing CV3 for convolving period-dimension data will be described. FIG. 10 is a schematic diagram illustrating spatial convolution processing CV1, phase convolution processing CV2, and period convolution processing CV3. In FIG. 10, first horizontal axis HA1 represents period, and second horizontal axis HA2 represents phase.

FIG. 10 shows an example of period-based skeleton data SKD items (i) whose phases are aligned and period-based skeleton data SKD items (i + 1) whose phases are aligned. Period-based skeleton data SKD items (i) indicate period-based skeleton data SKD items of period (i) extracted as the i-th period from a skeleton sequence. Period-based skeleton data SKD items (i + 1) indicate period-based skeleton data SKD items of period (i + 1) extracted as the i +1-th period from the skeleton sequence. Both period-based skeleton data SKD items (i) and period-based skeleton data SKD items (i + 1) include skeletons SK of specific phases. Here, using period-based skeleton data SKD items (i) and period-based skeleton data SKD items (i + 1) as examples, processing for convolving all joints J constituting skeleton SK will be described.

Spatial convolution processing CV1 indicates processing for convolving all joints J included in the same skeleton SK. Here, the same skeleton SK refers to skeleton SK of the same frame. Accordingly, all joints J are convolved for each skeleton SK of each phase of period (i), and the features of the spatial positional relationships among all joints J are extracted. Likewise, all joints J are convolved for each skeleton SK of each phase of period (i + 1), and the features of the spatial positional relationships among all joints J are extracted.

Phase convolution processing CV2 indicates processing for convolving each joint J (the same joint J) of the same period along the phase dimension. For example, joint J7 included in each phase of period (i) are convolved along the phase dimension. Likewise, joint J7 included in each phase of period (i + 1) are convolved along the phase dimension. Other joints J constituting skeleton SK are convolved in the same manner.

Period convolution processing CV3 indicates processing for convolving each joint J (the same joint J) corresponding to the same phase along the period dimension. For example, joint J8 of period (i) and joint J8 of period (i + 1) corresponding to the same phase are convolved along the period dimension. Other joints J constituting skeleton SK are convolved in the same manner.

According to the present embodiment, motion of each joint J can be convolved along the phase dimension. Moreover, motion of each joint J can be convolved along the period dimension. Accordingly, periodic features of motion of each joint J can be extracted. Therefore, periodic features of motion performed by subject SJ to be evaluated can be extracted.

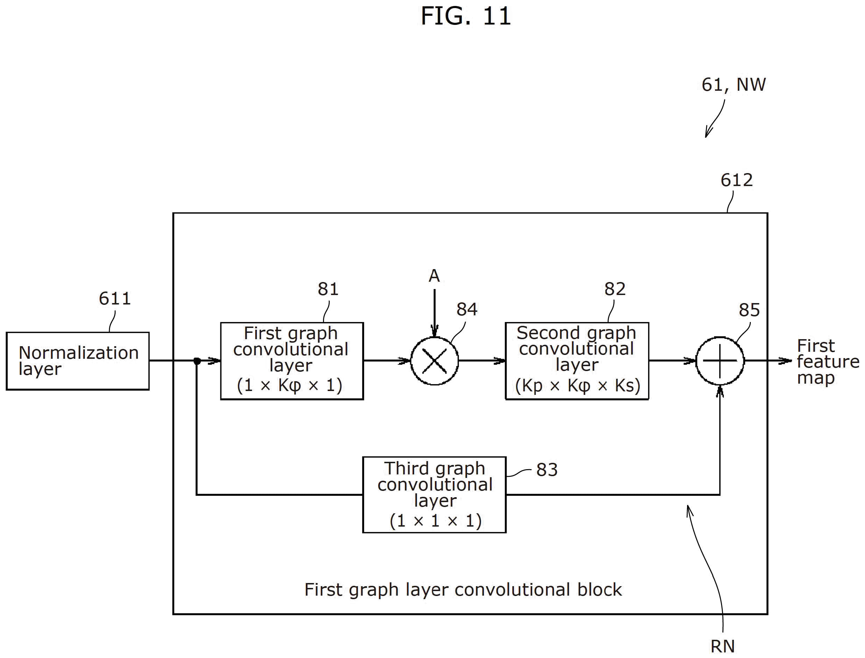

Next, one example of a network structure of first graph convolutional block 612 will be described with reference to FIG. 11. FIG. 11 is a diagram illustrating a neural network of first graph convolutional block 612 included in cognitive function evaluation system 100 according to the present embodiment. As shown in FIG. 11, first graph convolutional block 612 may include first graph convolutional layer 81, second graph convolutional layer 82, third graph convolutional layer 83, multiplier 84, and adder 85.

First input data (period-based joint dataset for each joint J) normalized by normalization layer 611 is input to first graph convolutional layer 81. First graph convolutional layer 81 generates a feature map by convolving the phase-dimension data included in the first input data. In other words, first graph convolutional layer 81 performs phase convolution processing CV2 described with reference to FIG. 10. First graph convolutional layer 81 is one example of a "first convolutional layer".

For example, first graph convolutional layer 81 may include three-dimensional graph convolutional layer having the kernel size of "1 × Kφ × 1". By using the three-dimensional graph convolutional layer having the size of "1 × Kφ × 1", the number of channels for performing operation on the spatial-dimension data and period-dimension data contained in the first input data is reduced. With this, the calculation number of convolution operations can be reduced.

Multiplier 84 multiplies the feature map output from first graph convolutional layer 81 by adjacency matrix A. Adjacency matrix A indicates spatial positional relationships (in the present embodiment, two-dimensional positional relationships) among joints J constituting skeleton SK. In the present embodiment, the period-based joint dataset for each joint J is input to first graph convolutional block 612. By multiplier 84 adding positional relationship information of each joint J to the period-based joint dataset of each joint J, spatial convolution processing CV1 and period convolution processing CV3 described with reference to FIG. 10 can be more accurately performed.

The first input data obtained after the phase-dimension data is convolved by first graph convolutional layer 81 is input to second graph convolutional layer 82. In the present embodiment, the output of multiplier 84 (the feature map to which the positional relationships among joints J are added) is input. Second graph convolutional layer 82 simultaneously performs, on the data input from multiplier 84, spatial convolution processing CV1, phase convolution processing CV2, and period convolution processing CV3 described with reference to FIG. 10. In other words, second graph convolutional layer 82 extracts a motion feature of subject SJ to be evaluated by simultaneously convolving the spatial-dimension data, phase-dimension data, and period-dimension data included in the first input data obtained after the phase-dimension data is convolved by first graph convolutional layer 81. More specifically, second graph convolutional layer 82 outputs a feature map indicating the motion feature of subject SJ to be evaluated. For example, first graph convolutional layer 81 may include three-dimensional graph convolutional layer having the kernel size of "Kp × Kφ × Ks". Second graph convolutional layer 82 is one example of a "second convolutional layer".

In the present embodiment, the neural network of first graph convolutional block 612 includes residual neural network (ResNet) RN. Specifically, third graph convolutional layer 83 and adder 85 constitute residual neural network RN. Residual neural network RN performs operation on a difference (residual) between the output of second graph convolutional layer 82 and first input data, and the operation result is output from first graph convolutional block 612 as the first feature map.

In detail, the first input data (period-based joint dataset for each joint J) normalized by normalization layer 611 is input to third graph convolutional layer 83. For third graph convolutional layer 83, a three-dimensional graph convolutional layer having the kernel size of "1 × 1 × 1" is used. Adder 85 generates the first feature map by adding the output (a feature map) of second graph convolutional layer 82 to the output (a feature map) of third graph convolutional layer 83. As a result, the first feature map is output from first graph convolutional block 612. Note that third graph convolutional layer 83 is one example of a "third convolutional layer".

According to the present embodiment, the inclusion of residual neural network RN in the neural network of first graph convolutional block 612 reduces the likelihood of gradient vanishing. With this, the training efficiency can be improved. Moreover, according to the present embodiment, third graph convolutional layer 83 having the kernel size of "1 × 1 × 1" reduces the number of dimensions of a feature map added to the output of second graph convolutional layer 82 (dimension reduction). Accordingly, the efficiency of operation processing in first graph convolutional block 612 can be improved.

Note that since second graph convolutional block 613 and third graph convolutional block 614 have network configurations substantially the same as the network configuration of first graph convolutional block 612, description thereof is omitted.

As described hereinbefore with reference to FIG. 11, the first input data obtained after the phase is convolved by first graph convolutional layer 81 is input to second graph convolutional layer 82. Accordingly, the amount of data processed in second graph convolutional layer 82 is reduced. With this, the number of operations performed by second graph convolutional layer 82 can be reduced.

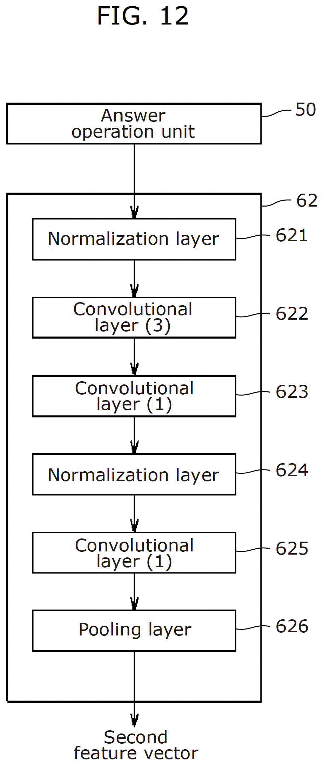

Next, one example of a network structure of answer feature extraction device 62 will be described with reference to FIG. 12. FIG. 12 is a diagram illustrating a neural network of answer feature extraction device 62 included in cognitive function evaluation system 100 according to the present embodiment. Answer feature extraction device 62 shown in FIG. 12 includes normalization layer 621, convolutional layer 622, convolutional layer 623, normalization layer 624, convolutional layer 625, and pooling layer 626. Normalization layer 621, convolutional layer 622, convolutional layer 623, normalization layer 624, convolutional layer 625, and pooling layer 626 are connected in series in the stated order.

The second input data is input to normalization layer 621 from answer operation unit 50. Normalization layer 621 normalizes the second input data. Normalization layer 621 includes, for example, a one-dimensional batch normalization layer.

The second input data normalized by normalization layer 621 is successively convolved by convolutional layer 622 and convolutional layer 623. Convolutional layer 622 includes, for example, a one-dimensional convolutional layer having a kernel size of three. Convolutional layer 623 convolves the output (a feature map) of convolutional layer 622 in the depth direction (depthwise convolution). For example, convolutional layer 623 includes a one-dimensional convolutional layer that convolves the output of convolutional layer 622 in the depth direction.

Normalization layer 624 normalizes the output (a feature map) of convolutional layer 623. Normalization layer 624 includes, for example, a one-dimensional batch normalization layer. In the similar manner of convolutional layer 623, convolutional layer 625 convolves the output of normalization layer 624 in the depth direction. For example, convolutional layer 625 includes a one-dimensional convolutional layer that convolves the output of normalization layer 624 in the depth direction.

The feature map is input to pooling layer 626 from convolutional layer 625. Pooling layer 626 converts the feature map into a feature vector (a second feature vector). Pooling layer 626 may include, for example, a global average pooling layer. Pooling layer 626 converts, for example, the feature map into a feature vector having the vector length of 160.

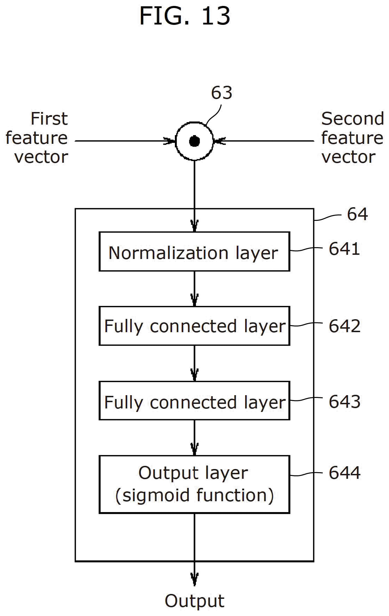

Next, one example of a network structure of fusion device 64 will be described with reference to FIG. 13. FIG. 13 is a diagram illustrating a neural network of fusion device 64 included in cognitive function evaluation system 100 according to the present embodiment. Fusion device 64 shown in FIG. 13 includes normalization layer 641, fully connected layer 642, fully connected layer 643, and output layer 644. Normalization layer 641, fully connected layer 642, fully connected layer 643, and output layer 644 are connected in series in the stated order.

A third feature vector is input to normalization layer 641 from coupling device 63. As has been already described, coupling device 63 generates the third feature vector by coupling the first feature vector and the second feature vector together. Normalization layer 641 normalizes the third feature vector. Normalization layer 641 includes, for example, a one-dimensional batch normalization layer.

The normalized third feature vector is input to output layer 644 after being linearly coupled (linearly transformed) by fully connected layer 642 and fully connected layer 643. By fully connected layer 642 and fully connected layer 643, the motion feature and answer feature of subject SJ to be evaluated are fused together. Output layer 644 outputs evaluation data (data showing an evaluation result of the cognitive function of subject SJ to be evaluated) based on the output of fully connected layer 643. Specifically, output layer 644 performs operation on the output of fully connected layer 643 based on a sigmoid function, and outputs data showing a predicted probability of being positive.

Note that when machine learning model ML is trained, processing unit 101 may use Binary Cross Entropy (BCE) loss to determine values of a plurality of parameters included in the neural network forming fusion device 64. Hereinafter, the values of the plurality of parameters included in the neural network forming fusion device 64 may be referred to as "parameter values of fusion device 64".

Binary Cross Entropy loss L_{BCE} is a loss function that minimizes the difference between a predicted probability distribution and a correct answer probability distribution. Accordingly, by adjusting a parameter value of fusion device 64 using Binary Cross Entropy loss L_{BCE}, the cognitive function of subject SJ to be evaluated can be more accurately classified. In detail, processing unit 101 adjusts a parameter value of fusion device 64 such that the value (loss value) of Binary Cross Entropy loss L_{BCE} is minimized.

Next, one example of loss function L used during the training of machine learning model ML will be described. In the present embodiment, when machine learning model ML is trained, loss function L in which first loss function Lₛₐₘₑ, second loss function Lₒₚₚ, and Binary Cross Entropy loss L_{BCE} are combined is used. When machine learning model ML is trained, processing unit 101 determines values of a plurality of parameters (parameter values) included in the neural network forming motion feature extraction device 61 using first loss function Lₛₐₘₑ and second loss function Lₒₚₚ. Hereinafter, values of a plurality of parameters included in the neural network forming motion feature extraction device 61 may be referred to as "parameter values of motion feature extraction device 61".

In detail, by calculating first loss function Lₛₐₘₑ and second loss function Lₒₚₚ, processing unit 101 determines a parameter value of motion feature extraction device 61 such that (i) first feature vectors generated from first input data items (explanatory variables) given the same label data (target variable) become more similar feature vectors and (ii) first feature vectors generated from first input data items (explanatory variables) given different label data items (target variables) become different feature vectors. First loss function Lₛₐₘₑ and second loss function Lₒₚₚ are loss functions for adjusting the parameter values of motion feature extraction device 61 such that similar first feature vectors are generated for samples of the same class and different first feature vectors are generated for samples of different classes.

Hereinafter, first feature vectors generated from first input data items given the same label data may be referred to as "first feature vectors of the same label data". Likewise, first feature vectors generated from first input data items given different label data items may be referred to as "first feature vectors of different label data items".

Specifically, first loss function Lₛₐₘₑ is a loss function that minimizes a cosine distance (pairwise cosine distance) between first feature vectors of the same label data. When machine learning model ML is trained, processing unit 101 calculates first loss function Lₛₐₘₑ for each of batches to adjust the parameter values of motion feature extraction device 61 such that the cosine distance between first feature vectors of the same label data is minimized. In other words, processing unit 101 adjusts a parameter value of motion feature extraction device 61 such that the value (loss value) of first loss function Lₛₐₘₑ is minimized. Accordingly, the parameter values of motion feature extraction device 61 are adjusted such that first feature vectors of the same label data become much more similar feature vectors. As a result, the classification accuracy of the cognitive function of subject to be evaluated SJ is improved.

In detail, during batch learning, processing unit 101 divides training data items corresponding to respective first feature vectors into a positive F+ sub-batch and a negative F- sub-batch based on label data items (correct answer labels). Next, processing unit 101 calculates loss Lₛₐₘₑ+ (a loss value) of the positive F+ sub-batch and loss Lₛₐₘₑ- (a loss value) of the negative F- sub-batch based on, for example, the following expressions (8) and (9).
[Math. 5] $\begin{matrix}{L}_{same}^{+} = {\sum }_{i} {\sum }_{j} 1 - \frac{{F}_{i}^{+} {F}_{j}^{+}}{\left‖{F}_{i}^{+}\right‖ \left‖{F}_{j}^{+}\right‖} \\ i , j ∈ \left[0, \left‖{F}^{+}\right‖\right] and i \neq j\end{matrix}$ (8)
[Math. 6] $\begin{matrix}{L}_{same}^{-} = {\sum }_{i} {\sum }_{j} 1 - \frac{{F}_{i}^{-} {F}_{j}^{-}}{\left‖{F}_{i}^{+}\right‖ \left‖{F}_{j}^{+}\right‖} \\ i , j ∈ \left[0, \left‖{F}^{-}\right‖\right] and i \neq j\end{matrix}$ (9)

Lastly, processing unit 101 calculates first loss function Lₛₐₘₑ. For example, first loss function Lₛₐₘₑ may be calculated based on the following expression (10). By calculating first loss function Lₛₐₘₑ shown by expression (10), a value (a loss value) obtained by weighting and summing loss Lₛₐₘₑ+ (a loss value) of the positive F+ sub-batch and Lₛₐₘₑ- (a loss value) of the negative F- sub-batch can be obtained.
[Math. 7] ${L}_{same} = \frac{1}{\left‖{F}^{+}\right‖ \left(\left‖{F}^{+}\right‖-1\right)} {L}_{same}^{+} + \frac{1}{\left‖{F}^{-}\right‖ \left(\left‖{F}^{-}\right‖-1\right)} {L}_{same}^{-}$ (10)

Next, second loss function Lₒₚₚ will be described. Second loss function Lₒₚₚ is a loss function that minimizes cosine similarity (pairwise cosine similarity) between first feature vectors of different label data items. When machine learning model ML is trained, processing unit 101 calculates second loss function Lₒₚₚ for each batch to adjust parameter values of motion feature extraction device 61 such that the cosine similarity between the first feature vectors of different label data items is minimized. In other words, processing unit 101 adjusts the parameter values of motion feature extraction device 61 such that the value (a loss value) of second loss function Lₒₚₚ is minimized. Accordingly, the parameter values of motion feature extraction device 61 are adjusted such that first feature vectors of different label data items become different feature vectors. As a result, the classification accuracy of the cognitive function of subject to be evaluated SJ is improved. For example, second loss function Lₒₚₚ may be calculated based on the following expression (11).
[Math. 8] $\begin{matrix}{L}_{opp} = \frac{1}{\left‖{F}^{+}\right‖ \left‖{F}^{-}\right‖} {\sum }_{i} {\sum }_{j} \frac{{F}_{i}^{+} {F}_{j}^{1}}{\left‖{F}_{i}^{+}\right‖ \left‖{F}_{j}^{-}\right‖} \\ i ∈ \left[1, \left‖{F}^{+}\right‖\right] , j ∈ \left[1, \left‖{F}^{-}\right‖\right]\end{matrix}$ (11)

Note that processing unit 101 may store a plurality of first feature vectors used for calculation of loss (loss value calculation) of (n - 1)-th batch and may use the stored first feature vectors again for loss calculation when n-th batch does not include one of either positive samples or negative samples. As a result, since the loss is always calculated even if a dataset used for training machine learning model ML is imbalanced, errors in batch processing can be avoided.

Moreover, prior to the training of machine learning model ML, an operator confirms that the first batch includes at least one positive sample and one negative sample. As a result, since a plurality of first feature vectors used for loss calculation in the first batch including the positive sample and the negative sample are stored, errors in batch processing can be avoided.

Next, loss function L will be described. As has been already described, loss function L is a loss function in which first loss function Lₛₐₘₑ, second loss function Lₒₚₚ, and Binary Cross Entropy loss L_{BCE} are combined. As has been already described, Binary Cross Entropy loss L_{BCE} is used for adjusting a parameter value of fusion device 64.

When machine learning model ML is trained, processing unit 101 calculates loss function L for each batch. As a result, for each batch, first loss function Lₛₐₘₑ, second loss function Lₒₚₚ, and Binary Cross Entropy loss L_{BCE} are calculated. For example, loss function L may be calculated based on the following expression (12). Note that in expression (12), "α" denotes the coefficient of first loss function Lₛₐₘₑ and "β" denote the coefficient of second loss function Lₒₚₚ.
[Math. 9] $L = \frac{1}{2 α + 2 β} \left(α+β\right) {L}_{BCE} + {αL}_{same} + {βL}_{opp}$ (12)

As shown by expression (12),weighting Binary Cross Entropy loss L_{BCE} by "α + β", i.e., the total value of α and β, enhances the effect of Binary Cross Entropy loss L_{BCE} to be greater than the effects of first loss function Lₛₐₘₑ and second loss function Lₒₚₚ. With this, the classification accuracy of the cognitive function can be improved. Moreover, "1 / (2α + 2β)" normalizes the magnitude of the total loss (loss value).

Hereinbefore, the embodiment of the present invention has been described with reference to FIG. 1 through FIG. 13. According to the present embodiment, the cognitive function of subject SJ to be evaluated can be evaluated using a dual-task. More specifically, the cognitive function of subject SJ to be evaluated can be evaluated based on periodicity of the motion (periodic motion feature) of subject SJ to be evaluated. Accordingly, the cognitive function of subject SJ to be evaluated can be more accurately evaluated.

In addition, according to the present embodiment, by evaluating the cognitive function of evaluation subject SJ based on the periodicity of the motion (periodic motion feature) of evaluation subject SJ, it is possible to avoid differences in individual physical abilities from affecting the evaluation result of the cognitive function. Accordingly, the cognitive function of subject SJ to be evaluated can be more accurately evaluated.

Furthermore, according to the present embodiment, the phases of periodic motion of subject SJ to be evaluated can be aligned. Accordingly, it is possible to avoid differences in the phases for each period affecting the evaluation of the cognitive function. As a result, the cognitive function of subject SJ to be evaluated can be more accurately evaluated.

Hereinbefore, the embodiment of the present invention has been described with reference to the drawings (FIG. 1 through FIG. 13). However, the present invention is not limited to the above-described embodiment, and may be implemented in various aspects without departing from the scope of the gist of the present invention. Moreover, the plurality of elements disclosed by the above-described embodiment can be modified as appropriate. For example, some elements among all the elements disclosed by an embodiment may be added to another embodiment, or some elements among the all elements disclosed by an embodiment may be deleted from the embodiment.

For ease of understanding the invention, the drawings schematically show the respective elements, and the thickness, length, number, spacing, and the like of each illustrated element may differ from the actual values for convenience in drawing the figures. In addition, the configuration of each element described in the above embodiment is one example and thus is not particularly limited. Accordingly, it is needless to say that various changes can be made within a scope not substantially departing from the advantageous effects produced by the present invention.

For example, in the embodiment described with reference to FIG. 1 through FIG. 13, the answer given by subject SJ to be evaluated performing a cognitive task and the answer given by subject SJ to be evaluated performing a dual-task are used for evaluating the cognitive function of subject SJ to be evaluated. However, evaluator 60 may extract an answer feature of only one answer out of the aforementioned answers.

Moreover, in the embodiment described with reference to FIG. 1 through FIG. 13, the answer given by subject SJ to be evaluated is used for evaluating the cognitive function of subject SJ to be evaluated, but the answer given by subject SJ to be evaluated may be omitted. In other words, the cognitive function of subject SJ to be evaluated may be evaluated from only the motion feature of subject SJ to be evaluated.

In addition, in the embodiment described with reference to FIG. 1 through FIG. 13, an imaging signal indicating motion of subject SJ to be evaluated performing a motor task is used for evaluating the cognitive function of subject SJ to be evaluated, but the imaging signal indicating the motion of subject SJ to be evaluated performing the motor task may be omitted.

Furthermore, in the embodiment described with reference to FIG. 1 through FIG. 13, subject SJ to be evaluated is caused to perform, once, a task set in which a cognitive task, a motor task, and a dual-task are sequentially assigned in the stated order, but subject SJ to be evaluated may be caused to sequentially perform the task set a plurality of times. In this case, first input data and second input data are generated for each task set. Motion feature extraction device 61 processes the first input data for each task set and extracts the motion feature of subject SJ to be evaluated for each task set. Likewise, answer feature extraction device 62 processes the second input data for each task set and extracts the answer feature of subject SJ to be evaluated for each task set. Accordingly, a first feature vector indicates the motion feature of subject SJ to be evaluated extracted for each task set, and a second feature vector indicates the answer feature of subject SJ to be evaluated extracted for each task set. As a result, when, for example, subject SJ to be evaluated is caused to sequentially perform a task set three times, the vector length of the first feature vector is "64 × 3" and the vector length of the second feature vector is "160 × 3". Accordingly, fusion device 64 fuses the first feature vector having a vector length of 192 (a 192-dimensional feature vector) and the second feature vector having a vector length of 480 (a 480-dimensional feature vector).

Moreover, in the embodiment described with reference to FIG. 1 through FIG. 13, a cognitive task, a motor task, and a dual-task are caused to be performed by subject SJ to be evaluated in the stated order, but the order in which the cognitive task, the motor task, and the dual-task are caused to be performed by subject SJ to be evaluated may be changed.

In addition, in the embodiment described with reference to FIG. 1 through FIG. 13, a cognitive task, a motor task, and a dual-task are caused to be performed by subject SJ to be evaluated, but the tasks to be performed by subject SJ to be evaluated may be tasks including at least a dual-task. For example, subject SJ to be evaluated may be caused to perform only a dual-task. Subject SJ to be evaluated may also be caused to perform a motor task and a dual-task or a cognitive task and a dual-task. Moreover, the task set to be performed by subject SJ to be evaluated may be a task that causes any one of a cognitive task, a motor task, or a dual-task to be performed two or more times. For example, the task set may include two or more dual-task items.

In addition, in the embodiment described with reference to FIG. 1 through FIG. 13, the cognitive function of subject SJ to be evaluated is classified into two classes, but evaluator 60 may classify the cognitive function of subject SJ to be evaluated into three or more classes. Specifically, evaluator 60 may classify the cognitive function of subject SJ to be evaluated into the class of dementia, the class of mild cognitive impairment, and the class of non-dementia. Alternatively, evaluator 60 may classify the cognitive function of subject SJ to be evaluated by the types of dementia. The types of dementia include Alzheimer-type dementia, vascular dementia, dementia with Lewy bodies, frontotemporal dementia, and normal pressure hydrocephalus, for example. For example, evaluator 60 may classify the cognitive function of subject SJ to be evaluated into the class of Alzheimer-type dementia, the class of dementia with Lewy bodies, the class of mild cognitive impairment, and the class of non-dementia.

Furthermore, in the embodiment described with reference to FIG. 1 through FIG. 13, the result of definitive diagnosis by a doctor is used for label data of training data, but the label data may be generated using the cognitive function score of subject SJ to be evaluated. For the cognitive function score, a typical intelligence evaluation scale may be used, such as a Mini-Mental State Examination (MMSE) score or Hasegawa's dementia scale-revised, for example. In diagnosis using an MMSE score, when the MMSE score is lower than or equal to 23, the subject is determined to be suspected of having dementia, and when the MMSE score is higher than 23 and lower than or equal to 27, the subject is determined to be suspected of having mild cognitive impairment. In addition, when the MMSE score is higher than 27, the subject is determined to be non-demented.

Moreover, in the embodiment described with reference to FIG. 1 through FIG. 13, evaluator 60 performs classification, but evaluator 60 may perform regression and classification. For example, evaluator 60 may determine the cognitive function score of subject SJ to be evaluated.

In addition, in the embodiment described with reference to FIG. 1 through FIG. 13, imager 10 includes a video camera or an RGB video camera, but imager 10 may include a depth camera. In this case, a motor feature of subject SJ to be evaluated may be extracted from a three-dimensional skeleton sequence.

Furthermore, in the embodiment described with reference to FIG. 1 through FIG. 13, skeleton SK includes 17 joints J, but the number of joints J constituting skeleton SK is not particularly limited provided that the whole body of subject SJ to be evaluated can be represented. For example, 25 joints J may constitute skeleton SK.

Moreover, in the embodiment described with reference to FIG. 1 through FIG. 13, skeleton generator 30 generates skeleton SK representing the whole body of subject SJ to be evaluated, but skeleton generator 30 may generate a skeleton representing a part of the whole body of subject SJ to be evaluated. For example, skeleton generator 30 may generate a skeleton representing the lower limbs of subject SJ to be evaluated. Skeleton generator 30 may also generate a skeleton representing the right-half or the left-half of subject SJ to be evaluated or may also generate a skeleton representing the knees, the lower back, or the like of subject SJ to be evaluated.

In addition, in the embodiment described with reference to FIG. 1 through FIG. 13, motion features of all joints J constituting skeleton SK are extracted, but motion features of some joints J among joints J constituting skeleton SK may be extracted.

Furthermore, in the embodiment described with reference to FIG. 1 through FIG. 13, each of the three convolutional blocks (first graph convolutional block 612, second graph convolutional block 613, and third graph convolutional block 614) includes residual neural network RN (third graph convolutional layer 83 and adder 85), but residual neural network RN (third graph convolutional layer 83 and adder 85) may be omitted.

Moreover, in the embodiment described with reference to FIG. 1 through FIG. 13, each of the three convolutional blocks (first graph convolutional block 612, second graph convolutional block 613, and third graph convolutional block 614) includes multiplier 84, but multiplier 84 may be omitted.

In addition, in the embodiment described with reference to FIG. 1 through FIG. 13, each of the three convolutional blocks (first graph convolutional block 612, second graph convolutional block 613, and third graph convolutional block 614) includes first graph convolutional layer 81, but first graph convolutional layer 81 may be omitted. For example, each of the three convolutional blocks (first graph convolutional block 612, second graph convolutional block 613, and third graph convolutional block 614) may include only second graph convolutional layer 82.

Furthermore, in the embodiment described with reference to FIG. 1 through FIG. 13, although motion feature extraction device 61 includes the three convolutional blocks (first graph convolutional block 612, second graph convolutional block 613, and third graph convolutional block 614), motion feature extraction device 61 may include one convolutional block (a graph convolutional block) or two or four or more convolutional blocks (graph convolutional blocks).

Moreover, in the embodiment described with reference to FIG. 1 through FIG. 13, task presenter 70 includes a display, but the configuration of task presenter 70 is not particularly limited provided that task presenter 70 can present a task to be performed by subject SJ to be evaluated. For example, task presenter 70 may include an audio output device.

In addition, in the embodiment described with reference to FIG. 1 through FIG. 13, answer detector 20 includes the left-hand answer switch and the right-hand answer switch, but the configuration of answer detector 20 is not particularly limited provided that answer detector 20 can detect an answer to a cognitive task. For example, answer detector 20 may include a gaze direction detection device or a sound collector.

When the gaze direction detection device is used, an answer given by subject SJ to be evaluated can be obtained based on, for example, the direction toward which subject SJ to be evaluated looked while answer-choice presentation screen 12b shown in part (b) of FIG. 3 and FIG. 4 is displayed. The gaze direction detection device can adopt a widely known gaze direction detection technique. For example, the gaze direction detection device includes near-infrared light-emitting diode (LED) and an imaging device. The near-infrared LED emits near-infrared light to the eye of subject SJ to be evaluated. The imaging device images the eye of subject SJ to be evaluated. Processing unit 101 analyzes an image captured by the imaging device, and detects the position of the pupil (direction of the gaze) of subject SJ to be evaluated.

When the sound collector is used, an answer given by subject SJ to be evaluated can be obtained based on, for example, a voice uttered by subject SJ to be evaluated toward answer-choice presentation screen 12b shown in part (b) of FIG. 3 and FIG. 4. For example, processing unit 101 can obtain the answer given by subject SJ to be evaluated by converting the voice uttered by subject SJ to be evaluated into text data through voice recognition processing.

Note that when the sound collector is used, a cognitive task is not limited to a test item in which subject SJ to be evaluated is caused to select one of two answer choices. For example, subject SJ to be evaluated may be caused to answer a calculation test item. Moreover, when the sound collector is used, a cognitive task may be a test item requiring a word as an answer. The test item requiring a word as an answer may be, for example, "a Shiritori (i.e., a word chain game) test item", "a test item of listing words (e.g., vocabulary words) that start with a sound (character) optionally selected from the Japanese syllabary", or "a test item of listing words (e.g., vocabulary words) that start with a character optionally selected from the alphabet".

### [Industrial Applicability]

The present invention can be used for diagnosis of dementia and mild cognitive impairment.

### [Reference Signs List]

- 10: imager
- 20: answer detector
- 30: skeleton generator (generator)
- 40: preprocessor
- 60: evaluator
- 81: first graph convolutional layer (first convolutional layer)
- 82: second graph convolutional layer (second convolutional layer)
- 84: multiplier
- 100: cognitive function evaluation system
- 612: first graph convolutional block (convolutional block)
- 613: second graph convolutional block (convolutional block)
- 614: third graph convolutional block (convolutional block)
- A: adjacency matrix
- CV1: spatial convolution processing
- CV2: phase convolution processing
- CV3: period convolution processing
- G1: time-series data of joint
- G2: period-based joint dataset
- G3: time-series data of joint
- G4: period-based joint dataset
- J: joint
- RN: residual neural network
- SJ: subject to be evaluated
- SK: skeleton
- SKD: period-based skeleton data

## Claims

1. A cognitive function evaluation system that evaluates a cognitive function of a subject to be evaluated, the cognitive function evaluation system comprising:
an imager that images the subject to be evaluated performing a dual-task in which a cognitive task and a motor task are simultaneously assigned, and generates imaging signals;
a generator that generates, based on the imaging signals, time-series data of a skeleton representing at least a part of a whole body of the subject to be evaluated performing the dual-task;
a preprocessor that decomposes the time-series data of the skeleton into periods to generate period-based skeleton data items, and aligns phases of the period-based skeleton data items to generate input data; and
an evaluator that extracts a motion feature of the subject to be evaluated based on the input data, and evaluates a cognitive function of the subject to be evaluated based on the motion feature of the subject to be evaluated which has been extracted.

2. The cognitive function evaluation system according to claim 1, wherein
the time-series data of the skeleton indicates time-series data of each of a plurality of joints included in the skeleton,
the plurality of joints indicate at least part of all joints included in the skeleton, and
the preprocessor decomposes the time-series data of each of the plurality of joints into periods to generate period-based joint data items, and aligns phases of the period-based joint data items to generate the input data.

3. The cognitive function evaluation system according to claim 2, wherein
from each of the period-based joint data items, the preprocessor samples a fixed number of data items of same phase points to generate the input data.

4. The cognitive function evaluation system according to claim 2 or 3, wherein
for each of the plurality of joints, the preprocessor selects, from among the period-based joint data items, a fixed number of consecutive period-based joint data items having a highest correlation to generate the input data.

5. The cognitive function evaluation system according to claim 2 or 3, wherein
the input data indicates the period-based joint data items whose phases are aligned,
the period-based joint data items whose phases are aligned indicate spatial-dimension data, phase-dimension data, and period-dimension data, and
the evaluator extracts the motion feature of the subject to be evaluated by convolving each of the spatial-dimension data, the phase-dimension data, and the period-dimension data.

6. The cognitive function evaluation system according to claim 5, wherein
the evaluator includes:
a first convolutional layer that convolves the phase-dimension data included in the input data; and
a second convolutional layer that extracts the motion feature of the subject to be evaluated by convolving each of the spatial-dimension data, the phase-dimension data, and the period-dimension data included in the input data obtained after the phase-dimension data is convolved by the first convolutional layer.

7. The cognitive function evaluation system according to claim 6, wherein
the evaluator further includes a multiplier that multiplies an adjacency matrix indicating positional relationships of the plurality of joints by an output of the first convolutional layer, and
an output of the multiplier is input to the second convolutional layer.

8. The cognitive function evaluation system according to claim 6, wherein
the evaluator further includes a residual neural network that performs operation on a difference between an output of the second convolutional layer and the input data.

9. The cognitive function evaluation system according to claim 5, wherein
the evaluator includes a plurality of convolutional blocks connected in series, and
each of the plurality of convolutional blocks convolves the spatial-dimension data, the phase-dimension data, and the period-dimension data.

10. The cognitive function evaluation system according to any one of claims 1 to 3, further comprising:
an answer detector that detects an answer given by the subject to be evaluated to a cognitive task, wherein
the answer detector detects at least one of (i) an answer given by the subject to be evaluated performing the dual-task or (ii) an answer given by the subject to be evaluated performing a cognitive task in which a cognitive task is assigned, and
the evaluator further extracts an answer feature of the answer detected by the answer detector, and evaluates the cognitive function of the subject to be evaluated, based on the motion feature and the answer feature of the subject to be evaluated.
